# EUROPEAN PATENT SPECIFICATION

(11) **EP 3 633 401 B1**
(45) Date of publication and mention of the grant of the patent: **12.08.2026**
(21) Application number: 18198623.3
(22) Date of filing: 04.10.2018
(51) Int. Cl.: G01R 33/565, G01R 33/567

(54) **PREVENTION OF COMPENSATING A WRONGLY DETECTED MOTION IN MRI**
VERHINDERN EINER KOMPENSATION EINER FÄLSCHLICHERWEISE ERFASSTEN BEWEGUNG IN DER MRT
PRÉVENTION DE COMPENSATION D'UN MOUVEMENT MAL DÉTECTÉ DANS L'IRM

(43) Date of publication of application: 08.04.2020
(73) Proprietor: Siemens Healthineers AG, 91301 Forchheim (DE)
(72) Inventor: Zeller, Mario, 91054 Erlangen (DE)
(74) Representative: Siemens Healthineers Patent Attorneys

(56) References cited:
- SINGH ADITYA ET AL: "Optical tracking with two markers for robust prospective motion correction for brain imaging", MAGNETIC RESONANCE MATERIALS IN PHYSICS, BIOLOGY AND MEDICINE, SPRINGER, DE, GB, vol. 28, no. 6, 30 June 2015 (2015-06-30), pages 523 - 534, XP035895954, ISSN: 0968-5243, [retrieved on 20150630], DOI: 10.1007/S10334-015-0493-4
- OOI M B ET AL: "Prospective real-time correction for arbitrary head motion using active markers", MAGNETIC RESONANCE IN MEDICINE, JOHN WILEY & SONS, INC, US, vol. 62, no. 4, 1 October 2009 (2009-10-01), pages 943 - 954, XP002602077, ISSN: 0740-3194, [retrieved on 20090601], DOI: 10.1002/MRM.22082

## Description

### Technical field

The present application relates to a method for detecting a wrongly detected motion of an object under examination during an MR imaging procedure, to a motion correction module, to a computer program comprising program code and to a computer readable data storage medium.

### Background

Magnetic Resonance Imaging is an imaging modality allowing a high-resolution generation of images of an object under examination such as a human being. Movements during an MR image acquisition, by of example the respiratory movements of the object under examination can result in artifacts, for example types known as ghosting, blurring and/or loss of intensity in the generated images. Numerous techniques are known for reducing artifacts as a result of respiratory motion, e.g. as disclosed in US 20160245888 A1, US 20170160367 A1, or US 20170160364 A1.

The publications SINGH ADITYA ET AL: "Optical tracking with two markers for robust prospective motion correction for brain imaging", MAGNETIC RESONANCE MATERIALS IN PHYSICS, BIOLOGY AND MEDICINE, SPRINGER, DE, GB, vol. 28, no. 6, 30 June 2015 (2015-06-30), pages 523-534 and Ooi M B ET AL: "Prospective real-time correction for arbitrary head motion using active markers", MAGNETIC RESONANCE IN MEDICINE, JOHN WILEY & SONS, 1 NC, US, vol. 62, no. 4, 1 October 2009 (2009-10-01), pages 943-954 disclose motion correction techniques using markers attached to the head of the patient.

The breathing of the person under examination can also be detected using external sensors such as a pneumatic cuff or based on signal processing of image signals generated by a camera monitoring the examined person. A camera based motion correction identifies the motion in the generated images of the camera and tries to correct the motion in the image. These camera based motion correction techniques can rely on a detection of a marker with a Moiré pattern mounted on top of a nose of the examined person whose head motion is supposed to be corrected, wherein the pattern is affected by a wrongly detected motion. This wrongly detected motion has the effect that the image quality of the generated motion compensated MR images that are generated based on the acquired MR signals, is often inferior to acquisitions without any motion correction.

The root cause of this effect seems to be twofold:
a) The examined person feels irritated by the marker sticking on the nose and thus slightly moves the nose, e.g. the alas of the nose while the head is not moving and remains still.
b) The alas of the nose, i.e. the nose itself is slightly moving during the inhaling or exhaling of the patient during respiration.

Since the camera detects the motion of the nose marker and assumes that the motion is rigid, the acquired raw data are wrongly corrected for the whole head. However, this problem does not only occur in the case where a marker is attached to the user's nose or head. Also in other motion correction techniques without the marker the head of the examined person is monitored and by post processing the detected images of the face of the person, the movement of the nose or any other part during inhaling or exhaling may be detected while the head itself which is examined is not moving at all.

Accordingly, a need exists to overcome the above-mentioned problems and to be able to differentiate a wrongly detected motion of an object under examination from the actual real motion of the object under examination during an acquisition of MR signals.

### Summary

This need is met by the features of the independent claims.

In the dependent claims further aspects of the invention are described.

According to a first aspect a method for separating a wrongly detected motion from an actual motion of a first part of an object under examination during an MR imaging procedure is provided at which MR signals from the first part of the object under examination are detected in order to generate MR images of the first part, wherein the actual motion influences the MR signal detection, and for detecting the wrongly detected motion of the first part of the object under examination in which the first part of the object under examination is not moving but a second part different from the first part of the object under examination is moving.

A first motion signal is detected of the object under examination during MR signal detection with a first sensor. Furthermore, a second motion signal of the object under examination during MR signal detection with a second sensor.

The first and second sensors are sensors which are not primarily used to generate the MR images, and the detected first motion signal and the detected second motion signal are independent of an acquisition of the MR signals used to generate the MR images. The moving of the second part is present in at least one of the two motion signals, and a presence of the actual and wrongly detected motion of the first part of the object under examination is different in the two motion signals.

The first motion signal is correlated with the second motion signal in order to separate the actual motion from the wrongly detected motion of the first part of the object under examination.

A motion compensation is carried out on the generated MR images wherein in the motion compensation substantially only the actual motion of the first part of the object under examination and not the wrongly detected motion of the first part of the object under examination is corrected in the detected MR signals.

With the detection of the two motion signals, it is possible to differentiate between an actual or real motion and the wrongly detected motion which occurs in a part of the object under examination but not in the part of the object where a major part of the MR images are generated from. As the first motion signal and the second motion signal are such that the wrongly detected motion may be present in both of the two signals, whereas the actual motion may be present in only one of the two signals, it is possible to differentiate between these two different motions. In another embodiment, one of the two motion signals carries or comprises the wrongly detected motion and the actual motion (thus both motion signals), whereas the other of the two motion signals carries either the wrongly detected or the actual motion. Thus the actual motion can be separated from the wrongly detected motion. It is possible to especially remove the signal influence from the respiratory motion in the detected motion signals during an MR head scan so that only the remaining motion, the real or actual motion is detected which can be used to carry out a motion compensation in the generated MR images.

The detected first motion signal and the detected second motion signal are independent of the acquisition of the MR signals used to generate the MR images. The detected first motion signal and the second motion signal can be detected during the entire examination after object under examination, the patient, and the signal detection of the motion signals works irrespectively of the fact whether an imaging sequence which is supposed to be corrected is currently running or whether adjustment data, localizer data or imaging data for a preceding measurement is acquired or the person examined is currently only instructed and no data is acquired at all. All these periods where no MR signals are detected for the MR images to be corrected, can be used for learning and continuously updating a correlation pattern generated between the second motion signal and the first motion signal. When the first and second motion signals are detected and if there is no analytical or empiric relation between the two motion signals, which is constant for the different image acquisitions, a learning phase may be introduced in which the relation between the two signals is learnt and which allows to separate the wrongly detected motion from the actual or real motion. This learning may happen continuously before the image acquisition for the image to be corrected is started. The first motion signal and the second motion signal can be signals detected from two different sensors configured to detect different signals of the object under examination. By way of example detecting one of the first motion signal or the second motion signal can comprise the acquisition of picture data of the object under examination with a camera and post processing the acquired picture data. The detecting of the first motion signal or second motion signal can also comprise acquiring sensor data from a sensor configured to detect the respiratory motion of the object under examination such as a respiratory sensor, e.g. a breathing belt etc..

Furthermore, for detecting the first motion signal or the second motion signal, it is possible to detect a pilot tone signal transmitted by a pilot tone transmitter, wherein this pilot tone signal is detected in different coil channels of at least one receiving coil used to detect the MR signals. This pilot tone signal is within a first frequency range which is detectable by the at least one receiving coil, but it is outside a second frequency range in which the MR signals are present and detected which are used to generate the MR images. By post processing the pilot tone signal of the different coil channels, it is possible to identify the wrongly detected motion and the actual motion.

By way of example the first motion signal contains the breathing curve as a surrogate that correlates with the wrongly detected motion e.g. occurring at the nose. The second signal originating from the coils signal channels from the pilot tone comprises in one channel the wrongly detected motion (of the nose) whereas the other channels only contain the true motion

When the pilot tone signal is used, correlating the first motion signal with the second motion signal, can mean that it is detected whether changes in the pilot tone signal are detected in the different channels of the at least one receiving coil. The actual motion can then be detected in the first motion signal or the second motion signal when the changes in the pilot tone signal are detected in all channels of the at least one receiving coil. When the changes are only detected on one or several but not all channels, one can deduce that the signal changes in the channel(s) where the changes are present are due to the wrongly detected motion.

In this context it is possible to detect the actual motion in the pilot tone signal as the first motion signal when the same changes of the pilot tone signal are detected in all sections of the at least one receiving coil. The wrongly detected motion is then present in the other motion signal, the second motion signal, but not in the first motion signal. The pilot tone can be a signal which can be generated by the MR system, or can be generated by a transmitter added to the receiving coil(s) or any other appropriate location near the MR system. The pilot tone has a frequency which is different from the signals and outside the frequency range used to excite the spins of the object under examination. Nevertheless, the pilot tone signal is modulated by the object under examination and can be detected by the receiving coils and if all the sections of the receiving coil or the receiving coils detect the same changes in the pilot tone signal, one can deduce that the object under examination in total has moved. If only a single part of the object under examination, such as the nose or the mouth is moving, changes in the pilot tone signal are not detected in all channels of the receiving coils.

The object under examination can be a head and the motion compensation can include the compensation of the rigid head movements occurring during the MR signal detection. Here, the head movement can include 6 degrees of freedom, three translational coordinates and three rotational coordinates.

The wrongly detected motion can be due to a respiration induced movement of a part of the nose present in both the first and second motion signals, wherein the actual motion signal is only present in one of the first and second motion signals. The respiration induced movement can thus be detected by correlating the two motion signals and can be removed in order to carry out the motion compensation only based on the actual rigid movement of the head.

Furthermore, a corresponding motion correction module according to claim 10 is provided

According to a further aspect a computer program according to claim 14 is provided

Additionally, a non-transitory, computer-readable data storage medium according to claim 15 is provided

It should be understood that the features mentioned above and features yet to be explained below can be used not only in the respective combinations indicated, but also in other combinations or in isolation, provided that the resulting subject-matter is within the scope of the appended claims.

### Brief description of the drawings

The foregoing and additional features and effects of the application will become apparent from the following detailed description, when read in conjunction with the accompanying drawings in which like reference numerals refer to like elements.
Figure 1 shows a schematic view of an MR system comprising a motion correction module configured to distinguish between wrongly detected motion and that actual motion of an object under examination.
Figure 2 shows a more detailed schematic view of a part of the system of Figure 1 in which a pilot tone signal detected in different channels of the receiving coil are used to differentiate a wrongly detected motion from an actual motion.
Figure 3 shows an example schematic view of a flowchart of a method carried out by a motion correction module used in the MR system of Figure 1.
Figure 4 shows an example schematic architecture view of the motion correction modular configured to detect a wrongly detected motion of an object during MR signal acquisition.

### Detailed description

In the following, embodiments of the invention will be described in detail with reference to the accompanying drawings. It is to be understood the following description of embodiments is not to be taken in a limiting sense. The scope of the invention is not intended to be limited by the embodiments described hereinafter or by the drawings, which are to be illustrative only.

The drawings are to be regarded as being schematic representations, and elements illustrated in the drawings are not necessarily shown to scale. Rather, the various elements are represented such that their function and general purpose becomes apparent to a person skilled in the art. Any connection or coupling between functional blocks, devices, components of physical or functional units shown in the drawings and described hereinafter may be implemented by an indirect connection or coupling. A coupling between components may be established over a wired or wireless connection. Functional blocks may be implemented in hardware, software, firmware, or a combination thereof.

In the following a procedure is explained in more detail how a wrongly detected motion can be detected in an examination of an object where MR signals are detected from the object under examination. The idea described below especially allows a differentiation between an actual motion which influences the MR signal detection and a motion of a part of the examined object, which substantially does not influence the detected MR signals or the generated MR images.

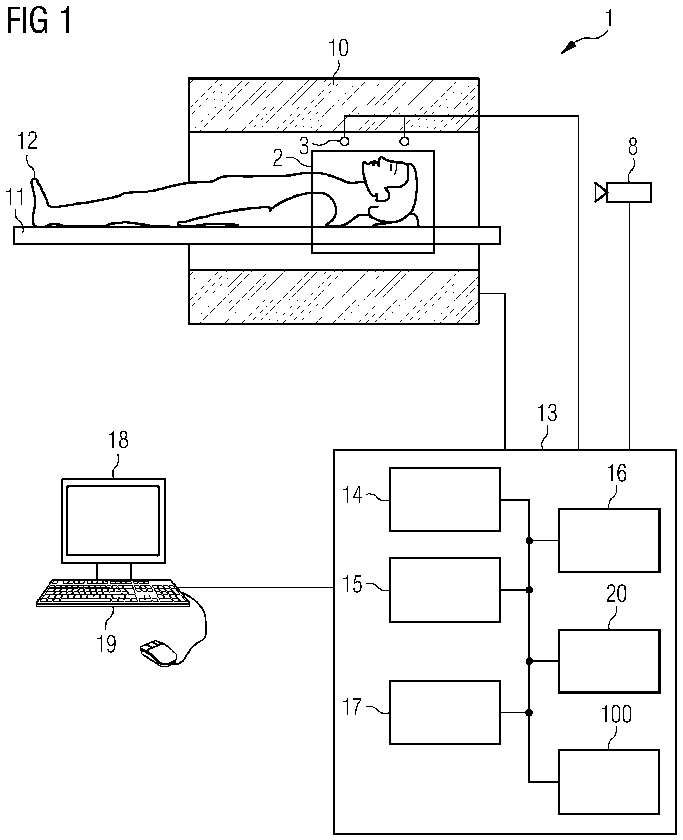

Figure 1 shows a schematic view of an MR system 1, which compromises a magnet 10 generating a polarization field B0. An object under examination 12 lying on a table 11 is moved into the center of the MR system 1 where MR signals after RF excitation can be detected by a receiving coil 2, which can comprise different coil sections wherein each coil section is associated with a corresponding detection channel. By applying RF pulses and magnetic field gradients, the nuclear spins in the object 12, especially in the part located in the receiving coil 2 are exited and the currents induced by the mechanization is detected. The way how MR images are generated and how the MR signals are detected using a sequence of RF pulses and the sequence of magnetic field gradients are known in the art so that a detailed explanation thereof is omitted.

The MR system comprises a control module 13 which is used for controlling the MR system. The control module 13 comprises a gradient control unit 14 for controlling and switching the magnetic field gradients, an RF control unit 15 for controlling and generating the RF pulses for the imaging sequences. An image sequence control unit 16 is provided which controls the sequence of the applied RF pulses and magnetic field gradients and thus controls the gradient control unit 14 and the RF control unit 15. In a memory 17, computer programs needed for operating the MR system and the imaging sequences necessary for generating the MR images can be stored together with the generated MR images. The generated MR images can be displayed on a display 18 wherein input unit 19 is provided used by a user of the MR system to control the functioning of the MR system. A processing unit 20 can coordinate the operation of the different functional units shown in Figure 1 and can comprise one or more processors which can carry out instructions stored on the memory 17. The memory includes the program code to be executed by the processing unit 20 or by a motion correction module 100 which is configured to correct the motion induced artifacts in the generated MR image. The motion correction module 100 is especially configured to differentiate between an actual motion of the object under examination, the person 12, and the wrongly detected motion which is a motion of the part of the examined object, which is however not the part which is mainly displayed in the generated MR images and from which the MR signals are detected. In Figure 1, furthermore a camera 8 is shown which is configured to acquire picture data from the object under examination. The generated picture data can be acquired with a frequency such that a movement of the part of the object under examination can be detected in the generated picture frames. By way of example, the frame rate of the generated picture frames may be between 1 and 50 frames per second.

The picture data can be post processed either by the processing unit 20 or by the motion correction module 100 in order to detect a motion of the person 12 during a time period when the MR signals are detected. Based on the acquired picture data a first motion signal can be generated which describes the motion of the person 12 during image acquisition.

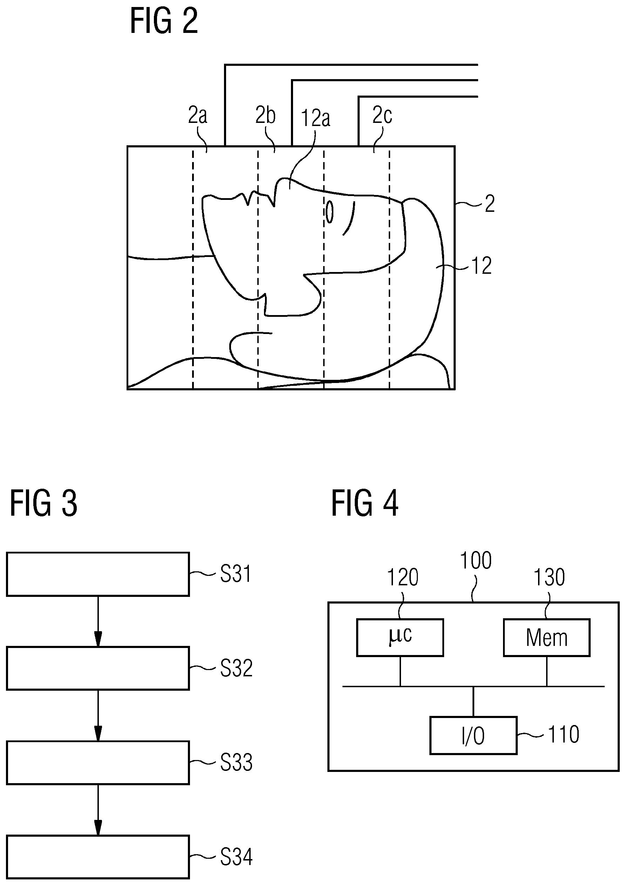

The RF control unit 15 can further be configured to act as a unit configured to generate a pilot tone which is transmitted by the MR system, either by a body coil (not shown) or by the indicated coil 2. This pilot tone signal is outside of the frequency range used for detecting the MR signal, but still within a frequency range that can be detected by the receiving coil, too. Further details about the generation and detection of pilot tone signals are disclosed in DE102015203385 A1. This pilot tone and the changes in the pilot tone occurring when the object under examination is moving can be used to generate a second motion signal which is deduced from the pilot tone signal that is detected in different coil channels of the receiving coil 2, as will be explained in more detail in connection with Figure 2.

A further option to determine a motion signal is based on a respiratory sensor such as a pneumatic cuff. This respiratory sensor is not shown in the figure for the sake of clarity.

Accordingly, the system uses at least two different sensors provided in the MR system 10 to distinguish between the actual and the wrongly detected motion of the object under examination. These two motion signals are independent of the detected MR signal and are especially independent of the position of the detected MR signal. The two sensors are sensors which are not primarily used to generate the MR images. Three different options for the motion sensors were discussed above, however it should be understood that other sensors such as radar sensors may be used to determine a movement or motion of any part of the object under examination. At least two of the motion signals generated by these sensors will be used to differentiate between the actual and the wrongly detected motion.

In connection with Figure 2, the receiving coil 2 with different coil channels 2a, 2b and 2c is shown. In each coil channel a separate MR image signal is detected, and the respiration of the patient 12 may lead to a movement of the alas 12a, shown in Fig. 2. A marker (not shown) may be mounted on top of the nose in order to improve the detection of the motion, however the marker is not necessarily provided as the motion may also be detected from the acquired picture data alone by a post processing the images and by detecting a movement of the patient.

In the following several options are disclosed how an actual movement of the head can be differentiated from a movement occurring only in a part of the nose which will be detected by the motion sensors discussed above, however which will have substantially no influence on the generated MR images of the head, especially of the interior part of the head.

First of all it is possible to use the breathing signal detected by a respiratory sensor, the pilot tone sender or the breathing belt to determine and mask out the periodically detected motion trajectory caused by the movement of the part of the face such as the nose which is substantially not present in the generated MR images. In this context it is possible to obtain the respiratory signal curve (first motion signal) and to obtain the x, y, z and the corresponding rotation angle is detected using the camera 8 (second motion signal). Additionally, it is possible to optionally correlate each coordinate with the respiratory signal curve during a learning phase and to store a similarity pattern. Over several breathing cycles the respiratory signal is correlated with each coordinate. When the correlation coefficient is similar over several time periods of the breathing cycle, it can be used and stored as reference coefficient for the corresponding coordinate. Later during MR signal acquisition the continuously determined correlation coefficients are determined. If they differ largely from the reference coefficient, one can deduce there is no respiratory influence on the motion signal and no motion correction is carried out. The motion signal averaged over several breathing cycles can be used to generate a model difference signal having low noise components which can be used to be subtracted from the wrongly detected motion signal. If needed the model difference signal can be scaled by a dynamically determined scaling coefficient. Coordinates for which the correlation is very low can be excluded from the processing. Thus each coordinate detected by the camera can be correlated with the respiratory signal curve during the imaging sequence and the correlation coefficients can be compared with previously stored ones. If the correlation is high and/or comparable to a similarity pattern calculated in an optional learning phase, it is possible to remove the signal portion due to the respiratory motion from the camera coordinates and to correct only the remaining portion. As mentioned above the wrongly detected motion can be present in both motion signals and the actual motion can be present in only one of the two signals.

In general any kind of correlation between the two motion signals can be used, the correlation can be any kind of correlation and can comprise a subtraction of one of the motion signals from the others, a quotient of the two signals, etc..

As an alternative, instead of using a respiratory sensor the pilot tone can be used which is detected in all the different coil elements 2a, 2b and 2c shown in Figure 2. The pilot tone as detected by the different coil segments is used to determine whether a motion trajectory detected by the camera is detected in all coil segments 2a, 2b or 2c, or only in some of the coil segments close to the nose. By way of example, if the motion is detected in all coil segments, it can be deduced that a rigid motion of the head is occurring which should be corrected. If, however, the main signal influence is occurring only in one or some of the coil segments such as coil segment 2b shown in Figure 2 one can deduce that the motion is mainly due to the motion of a part of the nose. This wrongly detected motion should not be used to correct the motion of the complete head as this would lead to a motion corrected image with a lower image quality compared to the non-motion compensated image.

In this implementation, the pilot tone signal may be detected from each coil segment, 2a, 2a and 2c. Furthermore, the translation and rotation coordinates are deduced from the camera data. Furthermore, it is possible to either directly determined the x, y, z and the respective rotation angles from the coil signals or to correlate each of the coil elements directly with the camera coordinates. Here it is possible to correlate the coordinates directly. In case of a high correlation, the camera signal is trusted and fully corrected. In case of a low correlation the correction is skipped until a high correlation is obtained. As an alternative it is possible to correlate the motion signal from the camera with the signal from the respective coil segments. In case of a similar correlation in all channels, the correction is carried out as this means that a rigid head movement occurs. In the case that the correlation is high only in the channel segments close to the nose or the mouth, the correction can be skipped until a uniform correlation is achieved again.

Another source for the wrongly detected motion is a case where the person to be examined moves the hand to the part of the body to be examined, e.g. for scratching, however the part of the patient to be imaged does not really move.

An advantage of the use of the different motion signals as discussed above is that they all receive data during the entire examination of the patient and work irrespective whether the imaging sequence which is supposed to be correct is currently running or whether adjustment data, localizer data or imaging data of another measurement are acquired or if no data is acquired at all.

A detection and modification of the motion signal can be performed for example by a thresholding, machine learning or a deep learning approach. Data of a previously acquired patient and feedback on the results by the MR system can be utilized to further improve the results. In a deep learning approach correction patterns for the motion correction can be deduced from existing data sets. Furthermore there are methods for evaluating an image quality. Motion signal graphs, for which the motion correction worked well, can be used as appropriate training data for the learning approach. Curves with which a poor motion correction quality was obtained, are used as "poor" training data. As an alternative to the automatic evaluation of images it is possible that a user, after a measurement, marks the data set as good or appropriate training data or as poor training data. Further if the MR image detection is repeated one might deduce that the former data set was a poor data set.

Figure 3 summarizes some of the main steps discussed above carried out by the motion correction module 100 shown in Figure 1. In step S31 a first motion signal of the object 12 is detected when the MR signal of the object 12 is detected. As discussed above, this first motion signal is independent of the position at which the MR signals are detected wherein the first motion signal can be a signal deduced from acquired picture data, can be a signal from a respiratory sensor such as a breathing belt or can be a signal deduced from the detected pilot tone signal. In step S32, the second motion signal is detected which is also independent of the acquired MR images. In connection with step S31, three different options are discussed for the motion signal. If one of these signals is used in step S31, another signal of the remaining options is used in step S32 to detect the second motion signal which is also independent of the acquisition of the MR signals. In step S33, the first motion signal and the second motion signal are correlated in order to differentiate a motion of the part of the object 12 which is actually shown in the generated MR images and a part of the object which substantially has no influence on the generated MR images. As the presence of the actual and the wrongly detected motion signal is different in both motion signals, a separation is possible. Then the actual motion is separated from the wrongly detected motion, it is possible to carry out a motion compensation, step S34, in which only the actual motion is compensated for.

Figure 4 shows a schematic architectural view of the motion correction module 100 shown in Figure 1. The motion correction module 100 can be a separate entity provided in the control module 13 or may be implemented as part of the processing unit 20 shown in Figure 1. If implemented as a separate entity, the motion correction module 100 comprises an interface or input/output 110 which is used to receive the first motion signal, a second motion signal and the picture data from the camera or any other data from the MR system or data from outside the MR system. The interface 110 is furthermore used to transmit data to the other entities such as the information about the differentiation between the actual and the wrongly detected motion. The entity furthermore comprises a processing unit 120 which is responsible for the operation of the motion correction module as discussed above. The processing unit can comprise one or more processors used to carry out instructions stored on a memory 130 wherein the memory 130 can be part of the memory 17 discussed above or can be a separate memory. The memory may include a read-only memory, a random access memory, mass storage, a hard disk or the like. The memory can furthermore include a suitable program code to be executed by the processing unit 120 so as to implement the above described functionalities in which the motion correction module is involved.

In summary different sensors are utilized which are incorporated into MR systems, wherein the data of the two motion sensors are correlated to improve the results of the sensor-based correction of movement. In general the image quality of the motion compensated image is improved so that more confidence is provided into the results.

## Claims

1. A method for separating a wrongly detected motion from an actual motion of a first part of an object under examination during an MR imaging procedure at which MR signals from the first part of the object under examination are detected in order to generate MR images of the first part, wherein the actual motion influences the MR signal detection and for detecting the wrongly detected motion of the first part of the object under examination in which the first part of the object under examination is not moving but a second part different from the first part of the object under examination is moving, the method comprising:
- detecting a first motion signal of the object under examination during MR signal detection, with a first sensor,
- detecting a second motion signal of the object under examination during MR signal detection, with a second sensor,
wherein the first and second sensors are sensors which are not primarily used to generate the MR images,
wherein the detected first motion signal and the detected second motion signal are independent of an acquisition of the MR signals used to generate the MR images,
wherein the moving of the second part is present in at least one of the two motion signals,
wherein a presence of the actual and wrongly detected motion of the first part of the object under examination is different in the two motion signals,
- correlating the first motion signal with the second motion signal in order to separate the actual motion from the wrongly detected motion of the first part of the object under examination,
- carrying out a motion compensation on the generated MR images wherein in the motion compensation substantially only the actual motion of the first part of the object under examination and not the wrongly detected motion of the first part of the object under examination is corrected in the detected MR signals.

2. The method according to claim 1, wherein detecting one of the first motion signal and the second motion signal comprises acquiring picture data of the object under examination with a camera and post-processing the acquired picture data.

3. The method according to any of the preceding claims, wherein detecting one of the first motion signal and the second motion signal comprises acquiring sensor data from a sensor configured to detect a respiratory motion of the object under examination.

4. The method according to any of the preceding claims, wherein detecting one of the first motion signal and the second motion signal comprises
- detecting a pilot tone signal transmitted by a pilot tone transmitter,
wherein the pilot tone signal is detected in different coil channels of at least one receiving coil used to detect the MR signals and is within a first frequency range which is detectable by the at least one receiving coil but outside a second frequency range in which the MR signals of the object under examination are present, and
- post-processing the pilot tone signal of the different coil channels.

5. The method according to claim 4,
wherein correlating the first motion signal with the second motion signal comprises detecting whether changes in the pilot tone signal are detected in the different channels of the at least one receiving coil,
wherein the actual motion is detected in either the first motion signal or the second motion signal, when the changes in the pilot tone signal are detected in all sections of the at least one receiving coil.

6. The method according to claim 4 or 5,
wherein the actual motion is detected in the pilot tone signal as first motion signal when the same changes of the pilot tone signal are detected in all channels of the at least one receiving coil,
wherein the wrongly detected motion is present in the second motion signal, but not in the first motion signal.

7. The method according to claim 5 or 6,
wherein the wrongly detected motion is determined based on the fact that the changes in the pilot tone signal are present in at least one but not in all channels of the at least one receiving coil.

8. The method according to any of the preceding claims,
wherein the object under examination is a head, and carrying out the motion compensation comprises compensating rigid head movements occurring during the MR signal detection.

9. The method according to claim 8,
wherein the wrongly detected motion is due to a respiration induced movement of a part of the nose present in both of the first and second motion signals,
wherein the actual motion is only present in one of the first and second motion signals,
wherein the respiration induced movement is removed in order to carry out the motion compensation only based on the actual rigid movement of the head.

10. A motion correction module configured to separate a wrongly detected motion from an actual motion of a first part of an object under examination during an MR imaging procedure at which MR signals from the first part of the object under examination are detected in order to generate MR images of the first part and to detect the wrongly detected motion of the first part of the object under examination in which the first part of the object under examination is not moving but a second part different from the first part of the object under examination is moving, the motion correction module comprising a first sensor and a second sensor, a memory and at least one processing unit, the memory containing instructions executable by said at least one processing unit, wherein the motion correction module is operative to:
- receive a first motion signal of the object under examination during MR signal detection, detected with the first sensor,
- receive a second motion signal of the object under examination during MR signal detection, detected with the second sensor, wherein the first and second sensors are sensors which are not primarily used to generate the MR images, such that the detected first motion signal and the detected second motion signal are independent of an acquisition of the MR signals used to generate the MR images,
- correlate the first motion signal with the second motion signal in order to separate the actual motion from the wrongly detected motion of the first part of the object under examination,
wherein the sensors are configured such that the moving of the second part is present in at least one of the two motion signals, and a presence of the actual and wrongly detected motion of the first part of the object under examination is different in the two motion signals,
- receive the MR signals detected from the first part of the object under examination,
- carry out a motion compensation on the generated MR signals, wherein in the motion compensation substantially only the actual motion of the first part of the object under examination and not the wrongly detected motion of the first part of the object under examination is corrected in the detected MR signals.

11. The motion correction module according to claim 10,
wherein the first or the second sensor comprises a camera, the motion correction module further being operative to carry out a method according to claim 2 and/or
wherein the first or the second sensor comprises a sensor configured to detect a respiratory motion of the object under examination, the motion correction module further being operative to carry out a method according to claim 3 and/or
wherein the first or the second sensor comprises a pilot tone transmitter and a receiving coil comprising different coil channels, the motion correction module further being operative to carry out a method according to any of claim 4 to 7.

12. The motion correction module according to claim 10 or 11,
wherein the object under examination is a head, and carrying out the motion compensation comprises compensating rigid head movements occurring during the MR signal detection.

13. The motion correction module according to claim 12,
wherein the wrongly detected motion is due to a respiration induced movement of a part of the nose present in both of the first and second motion signals,
wherein the actual motion is only present in one of the first and second motion signals,
wherein the motion correction module is operative to remove the respiration induced movement in order to carry out the motion compensation only based on the actual rigid movement of the head.

14. A computer program comprising program code to cause the module of claim 10 to execute the method of claim 1, to cause the module of claim 11 to execute a method according to any of claims 2-7, or to cause a module according to any of claims 10-13 to execute a method according to any of claims 8 or 9.

15. Non-transitory, computer-readable data storage medium comprising the computer program of claim 14.

## Patentansprüche

1. Verfahren zum Separieren einer falsch detektierten Bewegung von einer tatsächlichen Bewegung eines ersten Teils eines untersuchten Objekts während eines MR-Bildgebungsprozedur, bei der MR-Signale von dem ersten Teil des untersuchten Objekts detektiert werden, um MR-Bilder des ersten Teils zu erzeugen, wobei die tatsächliche Bewegung die MR-Signaldetektion beeinflusst, und zum Detektieren der falsch detektierten Bewegung des ersten Teils des untersuchten Objekts, wobei sich der erste Teil des untersuchten Objekts nicht bewegt, aber sich ein zweiter Teil, der sich von dem ersten Teil unterscheidet, des untersuchten Objekts bewegt, wobei das Verfahren Folgendes umfasst:
- Detektieren eines ersten Bewegungssignals des untersuchten Objekts während einer MR-Signaldetektion mit einem ersten Sensor,
- Detektieren eines zweiten Bewegungssignals des untersuchten Objekts während einer MR-Signaldetektion mit einem zweiten Sensor, wobei der erste und zweite Sensor Sensoren sind, die nicht primär zum Erzeugen der MR-Bilder verwendet werden,
wobei das detektierte erste Bewegungssignal und das detektierte zweite Bewegungssignal unabhängig von einer Erfassung der MR-Signale sind, die zum Erzeugen der MR-Bilder verwendet werden, wobei das Bewegen des zweiten Teils in wenigstens einem der zwei Bewegungssignale vorhanden ist,
wobei eine Anwesenheit der tatsächlichen und falsch detektierten Bewegung des ersten Teils des untersuchten Objekts in den zwei Bewegungssignalen unterschiedlich ist,
- Korrelieren des ersten Bewegungssignals mit dem zweiten Bewegungssignal, um die tatsächliche Bewegung von der falsch detektierten Bewegung des ersten Teils des untersuchten Objekts zu separieren;
- Ausführen einer Bewegungskompensation an den erzeugten MR-Bildern, wobei bei der Bewegungskompensation im Wesentlichen nur die tatsächliche Bewegung des ersten Teils des untersuchten Objekts und nicht die falsch detektierte Bewegung des ersten Teils des untersuchten Objekts in den detektierten MR-Signalen korrigiert wird.

2. Verfahren nach Anspruch 1, wobei das Detektieren von einem des ersten Bewegungssignals und des zweiten Bewegungssignals Erfassen von Bilddaten des untersuchten Objekts mit einer Kamera und Nachverarbeiten der erfassten Bilddaten umfasst.

3. Verfahren nach einem der vorhergehenden Ansprüche,
wobei das Detektieren von einem des ersten Bewegungssignals und des zweiten Bewegungssignals Erfassen von Sensordaten von einem Sensor umfasst, der dazu konfiguriert ist, eine Atembewegung des untersuchten Objekts zu detektieren.

4. Verfahren nach einem der vorhergehenden Ansprüche,
wobei das Detektieren von einem des ersten Bewegungssignals und des zweiten Bewegungssignals Folgendes umfasst:
- Detektieren eines Pilottonsignals, das durch einen Pilottonsender übertragen wird,
wobei das Pilottonsignal in verschiedenen Spulenkanälen von wenigstens einer Empfangsspule detektiert wird, die zum Detektieren der MR-Signale verwendet wird, und innerhalb eines ersten Frequenzbereichs liegt, der durch die wenigstens eine Empfangsspule detektierbar ist, aber außerhalb eines zweiten Frequenzbereichs liegt, in dem die MR-Signale des untersuchten Objekts vorhanden sind, und
- Nachverarbeiten des Pilottonsignals der verschiedenen Spulenkanäle.

5. Verfahren nach Anspruch 4,
wobei das Korrelieren des ersten Bewegungssignals mit dem zweiten Bewegungssignal Detektieren davon umfasst, ob Änderungen des Pilottonsignals in den verschiedenen Kanälen der wenigstens einen Empfangsspule detektiert werden,
wobei die tatsächliche Bewegung in entweder dem ersten Bewegungssignal oder dem zweiten Bewegungssignal detektiert wird, wenn die Änderungen des Pilottonsignals in allen Abschnitten der wenigstens einen Empfangsspule detektiert werden.

6. Verfahren nach Anspruch 4 oder 5,
wobei die tatsächliche Bewegung in dem Pilottonsignal als erstes Bewegungssignal detektiert wird, wenn die gleichen Änderungen des Pilottonsignals in allen Kanälen der wenigstens einen Empfangsspule detektiert werden,
wobei die falsch detektierte Bewegung in dem zweiten Bewegungssignal, aber nicht in dem ersten Bewegungssignal vorhanden ist.

7. Verfahren nach Anspruch 5 oder 6,
wobei die falsch detektierte Bewegung basierend auf der Tatsache bestimmt wird, dass die Änderungen des Pilottonsignals in wenigstens einem, aber nicht in allen Kanälen der wenigstens einen Empfangsspule vorhanden sind.

8. Verfahren nach einem der vorhergehenden Ansprüche,
wobei das untersuchte Objekt ein Kopf ist und das Ausführen der Bewegungskompensation Kompensieren starrer Kopfbewegungen umfasst, die während der MR-Signaldetektion auftreten.

9. Verfahren nach Anspruch 8,
wobei die falsch detektierte Bewegung auf eine atmungsinduzierte Bewegung eines Teils der Nase zurückzuführen ist, die sowohl in dem ersten als auch dem zweiten Bewegungssignal vorhanden ist, wobei die tatsächliche Bewegung nur in einem des ersten und zweiten Bewegungssignals vorhanden ist,
wobei die atmungsinduzierte Bewegung entfernt wird, um die Bewegungskompensation nur basierend auf der tatsächlichen starren Bewegung des Kopfes durchzuführen.

10. Bewegungskorrekturmodul, das dazu konfiguriert ist, eine falsch erfasste Bewegung von einer tatsächlichen Bewegung eines ersten Teils eines untersuchten Objekts während einer MR-Bildgebungsprozedur zu separieren, bei der MR-Signale von dem ersten Teil des untersuchten Objekts detektiert werden, um MR-Bilder des ersten Teils zu erzeugen, und die falsch detektierte Bewegung des ersten Teils des untersuchten Objekts zu detektieren, wobei sich der erste Teil des untersuchten Objekts nicht bewegt, aber sich ein zweiter Teil, der sich von dem ersten Teil unterscheidet, des untersuchten Objekts bewegt, wobei das Bewegungskorrekturmodul einen ersten Sensor und einen zweiten Sensor umfasst,
einen Speicher und wenigstens eine Verarbeitungseinheit, wobei der Speicher Anweisungen enthält, die durch die wenigstens eine Verarbeitungseinheit ausführbar sind, wobei das Bewegungskorrekturmodul zu Folgendem funktionsfähig ist:
- Empfangen eines ersten Bewegungssignals des untersuchten Objekts während einer MR-Signaldetektion, das mit dem ersten Sensor detektiert wird,
- Empfangen eines zweiten Bewegungssignals des untersuchten Objekts während einer MR-Signaldetektion, das mit dem zweiten Sensor detektiert wird, wobei der erste und zweite Sensor Sensoren sind, die nicht primär zum Erzeugen der MR-Bilder verwendet werden, so dass das detektierte erste Bewegungssignal und das detektierte zweite Bewegungssignal unabhängig von einer Erfassung der MR-Signale sind, die zum Erzeugen der MR-Bilder verwendet werden,
- Korrelieren des ersten Bewegungssignals mit dem zweiten Bewegungssignal, um die tatsächliche Bewegung von der falsch detektierten Bewegung des ersten Teils des untersuchten Objekts zu separieren;
wobei die Sensoren derart konfiguriert sind, dass das Bewegen des zweiten Teils in wenigstens einem der zwei Bewegungssignale vorhanden ist,
und eine Anwesenheit der tatsächlichen und falsch detektierten Bewegung des ersten Teils des untersuchten Objekts in den zwei Bewegungssignalen unterschiedlich ist,
- Empfangen der MR-Signale, die von dem ersten Teil des untersuchten Objekts detektiert werden,
- Ausführen einer Bewegungskompensation an den erzeugten MR-Signalen,
wobei bei der Bewegungskompensation im Wesentlichen nur die tatsächliche Bewegung des ersten Teils des untersuchten Objekts und nicht die falsch detektierte Bewegung des ersten Teils des untersuchten Objekts in den detektierten MR-Signalen korrigiert wird.

11. Bewegungskorrekturmodul nach Anspruch 10,
wobei der erste oder der zweite Sensor eine Kamera umfasst, wobei das Bewegungskorrekturmodul ferner zum Ausführen eines Verfahren nach Anspruch 2 funktionsfähig ist, und/oder
wobei der erste oder der zweite Sensor einen Sensor umfasst, der zum Detektieren einer Atmungsbewegung des untersuchten Objekts konfiguriert ist, wobei das Bewegungskorrekturmodul ferner zum Ausführen eines Verfahren nach Anspruch 3 funktionsfähig ist, und/oder
wobei der erste oder der zweite Sensor einen Pilottonsender und eine Empfangsspule umfasst, die verschiedene Spulenkanäle umfasst, wobei das Bewegungskorrekturmodul ferner dazu funktionsfähig ist, ein Verfahren nach einem der Ansprüche 4 bis 7 auszuführen.

12. Bewegungskorrekturmodul nach Anspruch 10 oder 11,
wobei das untersuchte Objekt ein Kopf ist und das Ausführen der Bewegungskompensation Kompensieren starrer Kopfbewegungen umfasst, die während der MR-Signaldetektion auftreten.

13. Bewegungskorrekturmodul nach Anspruch 12,
wobei die falsch detektierte Bewegung auf eine atmungsinduzierte Bewegung eines Teils der Nase zurückzuführen ist, die sowohl in dem ersten als auch dem zweiten Bewegungssignal vorhanden ist, wobei die tatsächliche Bewegung nur in einem des ersten und zweiten Bewegungssignals vorhanden ist,
wobei das Bewegungskorrekturmodul dazu funktionsfähig ist, die atmungsinduzierte Bewegung zu entfernen, um die Bewegungskompensation nur basierend auf der tatsächlichen starren Bewegung des Kopfes durchzuführen.

14. Computerprogramm, das Programmcode umfasst, um zu bewirken, dass das Modul nach Anspruch 10 das Verfahren nach Anspruch 1 ausführt, um zu bewirken, dass das Modul nach Anspruch 11 ein Verfahren nach einem der Ansprüche 2-7 ausführt, oder um zu bewirken, dass ein Modul nach einem der Ansprüche 10-13 ein Verfahren nach einem der Ansprüche 8 oder 9 ausführt.

15. Nichtflüchtiges computerlesbares Datenspeicherungsmedium, das das Computerprogramm nach Anspruch 14 umfasst.

## Revendications

1. Un procédé de séparation d'un mouvement faussement détecté d'un mouvement réel d'une première partie d'un objet en examen pendant une procédure d'imagerie RM, où des signaux RM provenant de la première partie de l'objet en examen sont détectés afin de créer des images RM de la première partie, dans lequel le mouvement réel influence la détection de signaux RM et de détecter le mouvement faussement détecté de la première partie de l'objet en examen, dans lequel la première partie de l'objet en examen n'est pas en déplacement, mais une deuxième partie, différente de la première partie, de l'objet en examen est déplacée, le procédé comprenant :
- détecter un premier signal de mouvement de l'objet en examen pendant une détection de signaux RM par un premier capteur,
- détecter un deuxième signal de mouvement de l'objet en examen pendant une détection de signaux RM par un deuxième capteur,
dans lequel le premier et le deuxième capteurs sont des capteurs, qui ne sont pas utilisés principalement pour créer les images RM, dans lequel le premier signal détecté de mouvement et le deuxième signal détecté de mouvement sont indépendants d'une acquisition des signaux RM utilisés pour créer les images RM,
dans lequel le déplacement de la deuxième partie est présent dans au moins l'un des deux signaux de mouvement,
dans lequel une présence du mouvement réel et du mouvement faussement détecté de la première partie de l'objet en examen est différente dans les deux signaux de mouvement,
- mettre le premier signal de mouvement en corrélation avec le deuxième signal de mouvement, afin de séparer le mouvement réel du mouvement faussement détecté sur la première partie de l'objet en examen,
- effectuer une compensation de mouvement sur les images RM créées, dans lequel, dans la compensation de mouvement, sensiblement seulement le mouvement réel de la première partie de l'objet en examen, et non le mouvement faussement détecté dans la première partie de l'objet en examen, est corrigé dans les signaux RM détectés.

2. Le procédé suivant la revendication 1, dans lequel détecter l'un du premier signal de mouvement et du deuxième signal de mouvement comprend acquérir une donnée d'image de l'objet en examen par une caméra et post-traiter la donnée d'image acquise.

3. Le procédé suivant l'une quelconque des revendications précédentes,
dans lequel détecter l'un du premier signal de mouvement et du deuxième signal de mouvement comprend acquérir une donnée de capteur d'un capteur configuré pour détecter un mouvement respiratoire de l'objet en examen.

4. Le procédé suivant l'une quelconque des revendications précédentes,
dans lequel détecter l'un du premier signal de mouvement et du deuxième signal de mouvement comprend
- détecter un signal de fréquence pilote émis par un émetteur de fréquence pilote,
dans lequel le signal de fréquence pilote est détecté dans des canaux différents d'au moins une bobine de réception utilisée pour détecter les signaux RM et est dans une première plage de fréquence, qui peut être détectée par la au moins une bobine de réception, mais en dehors d'une deuxième plage de fréquence, dans laquelle les signaux RM de l'objet en examen sont présents, et
- post-traiter le signal de fréquence pilote des différents canaux de la bobine.

5. Le procédé suivant la revendication 4,
dans lequel mettre le premier signal de mouvement en corrélation avec le deuxième signal de mouvement comprend détecter, si des changements dans le signal de fréquence pilote sont détectés dans les canaux différents de la au moins une bobine de réception,
dans lequel le mouvement réel est détecté soit dans le premier signal de mouvement, soit dans le deuxième signal de mouvement, lorsque les changements dans le signal de fréquence pilote sont détectés dans toutes les parties de la au moins une bobine de réception.

6. Le procédé suivant la revendication 4 ou 5,
dans lequel le mouvement réel est détecté dans le signal de fréquence pilote comme premier signal de mouvement, lorsque les mêmes changements du signal de fréquence pilote sont détectés dans tous les canaux de la au moins une bobine de réception,
dans lequel le mouvement faussement détecté est présent dans le deuxième signal de mouvement, mais non dans le premier signal de mouvement.

7. Le procédé suivant la revendication 5 ou 6,
dans lequel le mouvement faussement détecté est déterminé, sur la base du fait que les changements dans le signal de fréquence pilote sont présents dans au moins l'un, mais non dans tous les canaux de la au moins une bobine de réception.

8. Le procédé suivant l'une quelconque des revendications précédentes,
dans lequel l'objet en examen est une tête et effectuer la compensation de mouvement comprend compenser des mouvements rigides de la tête se produisant pendant la détection de signaux RM.

9. Le procédé suivant la revendication 8,
dans lequel le mouvement faussement détecté est dû à un mouvement induit par la respiration, d'une partie du nez présent à la fois dans le premier et le deuxième signal de mouvement,
dans lequel le mouvement réel est présent seulement dans l'un du premier et du deuxième signal de mouvement,
dans lequel le mouvement, induit par la respiration, est retiré, afin d'effectuer la compensation du mouvement, seulement sur la base du mouvement réel rigide de la tête.

10. Un module de correction de mouvement configuré pour séparer un mouvement faussement détecté d'un mouvement réel d'une première partie d'un objet en examen pendant une procédure d'imagerie RM, où des signaux RM provenant de la première partie de l'objet en examen sont détectés, afin de créer des images RM de la première partie et de détecter le mouvement faussement détecté de la première partie de l'objet en examen, dans lequel la première partie de l'objet en examen n'est pas en mouvement, mais une deuxième partie, différente de la première partie, de l'objet en examen est en mouvement, le module de correction de mouvement comprenant un premier capteur et un deuxième capteur,
une mémoire et au moins une unité de traitement, la mémoire contenant des instructions pouvant être exécutées par ladite au moins une unité de traitement, dans lequel le module de correction de mouvement fonctionne pour :
- recevoir un premier signal de mouvement de l'objet en examen pendant une détection de signaux RM détectée par le premier capteur,
- recevoir un deuxième signal de mouvement de l'objet en examen pendant une détection de signaux RM détectée par le deuxième capteur, dans lequel le premier et le deuxième capteurs sont des capteurs, qui ne sont pas utilisés principalement pour créer les images RM, de manière à ce que le premier signal détecté de mouvement et le deuxième signal détecté de mouvement soient indépendants d'une acquisition des signaux RM utilisés pour créer les images RM,
- mettre le premier signal de mouvement en corrélation avec le deuxième signal de mouvement, afin de séparer le mouvement réel du mouvement faussement détecté de la première partie de l'objet en examen,
dans lequel les capteurs sont configurés de manière à ce que le mouvement de la deuxième partie soit présent dans au moins l'un des deux signaux de mouvement,
et une présence du mouvement réel et faussement détecté de la première partie de l'objet en examen soit différente dans les deux signaux de mouvement,
- recevoir les signaux RM détectés provenant de la première partie de l'objet en examen,
- effectuer une compensation du mouvement sur les signaux RM créés, dans lequel, dans la composition de mouvement, sensiblement, seulement le mouvement réel de la première partie de l'objet en examen, et non le mouvement faussement détecté de la première partie de l'objet en examen, est corrigé dans les signaux RM détectés.

11. Le module de correction de mouvement suivant la revendication 10,
dans lequel le premier et le deuxième capteurs comprennent une caméra, le module de correction de mouvement fonctionnant, en outre, pour effectuer un procédé suivant la revendication 2 et/ou dans lequel le premier ou le deuxième capteur comprend un capteur configuré pour détecter un mouvement respiratoire de l'objet en examen, le module de correction de mouvement fonctionnant, en outre, pour effectuer un procédé suivant la revendication 3 et/ou dans lequel le premier et le deuxième capteurs comprennent un émetteur de fréquence pilote et une bobine de réception comprenant des canaux de bobine différents, le module de correction de mouvement fonctionnant, en outre, pour effectuer un procédé suivant l'une quelconque des revendications 4 à 7.

12. Le module de correction suivant la revendication 10 ou 11, dans lequel l'objet en examen est une tête, et effectuer la compensation de mouvement comprend compenser des mouvements rigides de la tête, se produisant pendant la détection de signaux RM.

13. Le module de correction suivant la revendication 12,
dans lequel le mouvement faussement détecté est dû à un mouvement induit par la respiration d'une partie du nez présent, dans à la fois le premier et le deuxième signal de mouvement,
dans lequel le mouvement réel est présent seulement dans l'un du premier et du deuxième signal de mouvement,
dans lequel le mouvement induit par la respiration est retiré afin d'effectuer la compensation du mouvement, seulement sur la base du mouvement réel rigide de la tête.

14. Un programme d'ordinateur, comprenant un code de programme pour faire que le module de la revendication 10 exécute le procédé de la revendication 1, pour faire que le module de la revendication 11 exécute un procédé suivant l'une quelconque des revendications 2 à 7, ou pour faire qu'un module suivant l'une quelconque des revendications 10 à 13 exécute un procédé suivant l'une quelconque des revendications 8 ou 9.

15. Support de mémoire de données non transitoire, déchiffrable par ordinateur, comprenant le programme d'ordinateur de la revendication 14.
